# EUROPEAN PATENT SPECIFICATION

(11) **EP 2 217 140 B1**
(45) Date of publication and mention of the grant of the patent: **08.02.2012**
(21) Application number: 08855824.2
(22) Date of filing: 26.11.2008
(51) Int. Cl.: A61B 5/024

(54) **APPARATUS AND METHOD FOR DETECTION OF SYNCOPES**
GERÄT UND VERFAHREN ZUM NACHWEIS VON SYNKOPEN
APPAREIL ET PROCÉDÉ DE DÉTECTION DE SYNCOPES

(30) Priority: 06.12.2007 EP 07122515
(43) Date of publication of application: 18.08.2010
(73) Proprietor: Koninklijke Philips Electronics N.V., 5621 BA Eindhoven (NL); Philips Intellectual Property & Standards GmbH, 20099 Hamburg (DE)
(72) Inventor: THIJS, Jeroen, A., J., NL-5656 AE Eindhoven (NL); MÜHLSTEFF, Jens, NL-5656 AE Eindhoven (NL)
(74) Representative: Van Velzen, Maaike Mathilde
(86) International application number: PCT/IB2008/054958
(87) International publication number: WO 2009/072034

(56) References cited:
- WO-A-2007/026281
- US-A1- 2007 070 800

## Description

### FIELD OF THE INVENTION

The invention relates to a method and apparatus for the detection of vasovagal syncopes.

### BACKGROUND OF THE INVENTION

A vasovagal syncope is a sudden loss of conciousness, the causes of which are not fully understood, but which involves a loss of blood pressure leading to fainting. In elderly patients, such syncopes are dangerous as fainting may lead to injuries from falls.

The population is aging and a large percentage of the population over seventy years of age will suffer a vasovagal syncope at some point over the next decade. One statistic suggests that 23% of the elderly population will suffer a syncope in ten years, and such events can account for 5% of emergency visits and 3% of hospital visits for such patients. The problems of vasovagal syncopes are accordingly widespread.

Such syncopes can represent a serious problem for elderly patients which makes it much more difficult for them to live independently.

It would be highly advantageous to provide apparatus to provide an advance warning of such a syncope.

A prior proposal is presented in US2007/0070800 which incorporates a photoplethysmographic sensor and estimates a probability of a syncope from the measured sensor signal.

An alternative solution is described in WO2002/41771 which measures blood pressure using a cuff.

### SUMMARY OF THE INVENTION

According to the invention there is provided apparatus according to claim 1.

By using the pulse transit time approach much more reliable results can be obtained than with the prior proposals mentioned above. The method is sufficiently simple that it can be incorporated into low power apparatus that can be permanently worn by the patient as the patient carries on their normal life.

Importantly, the approach adopted can give advance warning of an impending syncope and allow time for the patient to sit or lie down to minimise the risk of falling. In another aspect, the invention relates to a method according to claim 10.

### BRIEF DESCRIPTION OF THE DRAWINGS

For a better understanding of the invention embodiments will now be described with reference to the accompanying drawings in which:
Fig. 1 shows a patch;
Fig. 2 shows a wristband;
Fig. 3 shows a processing unit; and
Fig. 4 shows the steps of a method for detecting vasovagal syndrome.

The drawings are schematic and not to scale. Like components are given the same reference numerals in the different figures.

### DETAILED DESCRIPTION OF THE EMBODIMENTS

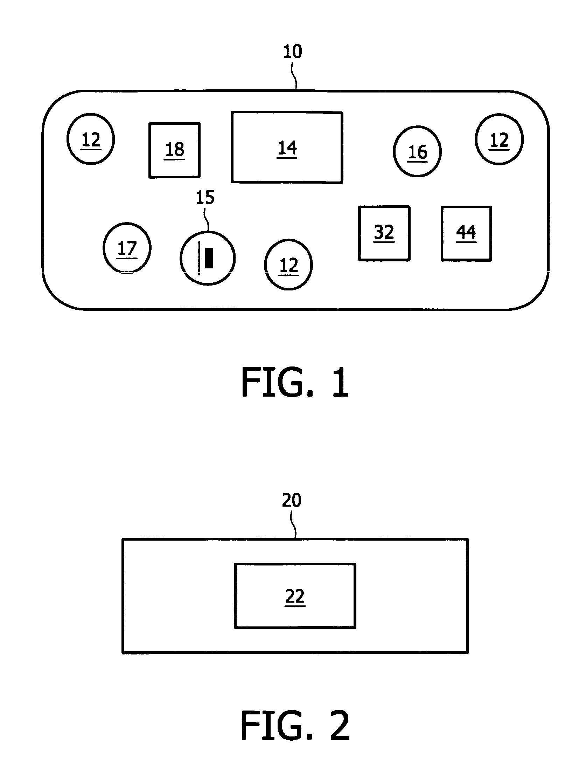

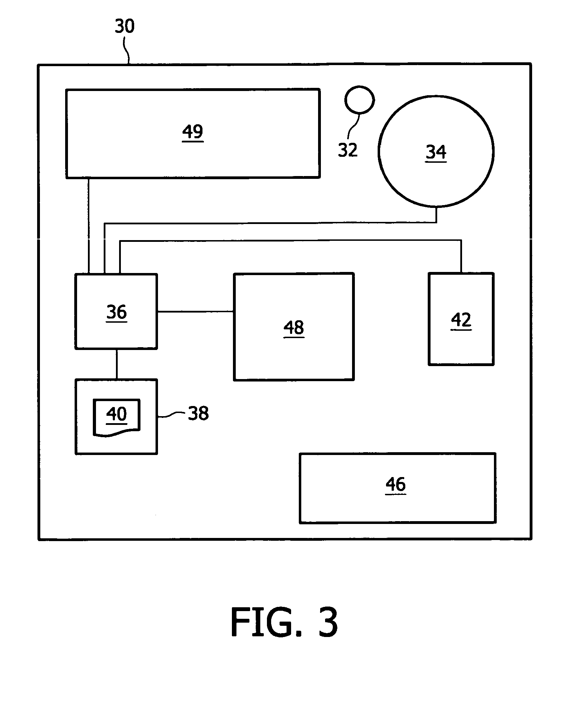

Referring to Figs. 1 to 3, an embodiment of the invention includes a patch 10, a wristband 20 and a processing unit 30. The patch 10 is worn on the chest and in the embodiment is incorporated in an item of apparel, here a T-shirt.

A number of sensors are incorporated into the patch 10. The patch 10 includes ECG sensors 12.

The patch 10 also includes a further sensor, an accelerometer 14 for measuring activity and posture. A battery 17 is also provided, and the whole patch should use a very low power consumption. Other features of the patch are optional and are discussed below.

The wrist band 20 includes a pulse sensor 22.

The pulse sensor 22 may be a mechanical piezo sensor, for example, an optical sensor measuring a change in light absorption to detect a passing pulse wave, or a bioimpedance sensor.

A processing unit 30 is provided and worn on the body. The processing unit 30 includes a microphone 32 for capturing speech of the patient and a loudspeaker 34. The processing unit also includes a processor 36 and a memory 38 including code 40 for controlling the processor. A transceiver 42 is provided for providing radio contact and in particular for contacting the emergency services if required. A display 49 is provided to output data and operational messages.

The processing unit includes a battery 46, and operates at low power to achieve lengthy battery life without needing an excessive battery weight.

A memory 48 is used for recording sensor data over a period of time, in order to enable a retrospective analysis of a patient's vital signs. The memory 48 may store data over a period of 24 hours or more.

In use, the sensors continuously capture hemodynamic parameters and pass them to the processing unit 30. The parameters include ECG and pulse transit times. The data is processed and stored in memory 48.

Data, including for example ECG data, may be recorded over a period. This allows data taken during a syncope event. Such data may be of use to a medical practitioner caring for a patient since diagnosis of a vasovagal syncope is otherwise very difficult if the syncope is not observed by the medical practitioner.

Existing tests for detecting syncopes use complex computer systems and continual monitoring. However, it is important that the processing can be carried out on a low power unit. Thus, prior approaches which use complex data processing to carry out the check are not suitable. Since the existing tests are not suitable, the inventors have designed a simple algorithm that can be implemented in low power devices with low processing capabilities.

In particular, the inventors use a combination of the pulse transit time approach and context data. The context data include time, information regarding posture and activity measured from the accelerometer 44, as well as recorded speech and noises from the patient.

An important parameter is thus the pulse transit time which is measured by detecting the transit time of a pulse between the patch 10 and the wrist band 20. The pulse at the patch 10 may be determined from the ECG data, for example the R-peak in the ECG. The pulse at the wrist band may be measured by any convenient pulse detection technique.

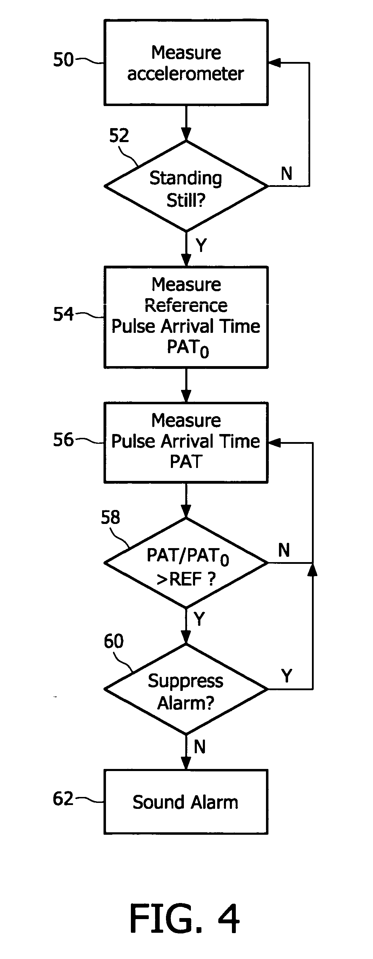

The simple algorithm used in the embodiment is as follows, as illustrated in Fig. 4. Firstly, the accelerometer is used (step 50) to detect posture change, and in particular to detect when the patient is stationary in a standing position (step 52). Then, as the patient stands still for a complete minute, the system measures a reference pulse transit time (PAT0), the time between a predetermined feature of the ECG pulse and the detection of the pulse at the pulse sensor (step 54).

Then, the system continues to monitor the pulse transit time in the same way (step 56). The ratio PAT/PAT0 of the measured pulse transit time (PAT) over the reference pulse transit time is calculated. When this ratio exceeds a predetermined limit (step 58), the context data is checked (step 60) to see if the patient is in a position or state for which the alarm is not to be sounded. In particular, the accelerometer data provides important context data. If the patient is relatively stationary and horizontal, i.e. asleep, there may be no need to sound the alarm which may accordingly be suppressed as unnecessary in this case.

If the ratio exceeds the limit, and the context data does not indicate that the alarm is unnecessary, the alarm sounds (step 62).

As will be appreciated, the code 40 is arranged to cause the processor 36 to carry out the steps of this method when the code is executed on the processor 36.

In an embodiment, the predetermined limit of the ratio PAT/PAT0 may be in the range 1.08 to 1.2.

This calculation is simple and hence does not overload the processing capability of the processing unit 30 yet is still capable of providing good advance warning for a vasovagal syncope.

The warning may take a number of forms. In the embodiment, a predetermined message is played on the loudspeaker. The message may be, for example, a message "lay down immediately! put your legs up". The message may also be a warning message intended for passers by and bystanders.

After playing the warning message, the processor in the embodiment continues to monitor the patient. In the event of the patient not recovering quickly enough from the syncope, or in the case additional help is required, the processor calls the emergency services by automatically calling a call centre using the transceiver. In particular, if the patient has fallen this is picked up from the accelerometer data in which case the emergency services will be called.

In the embodiment, the ECG sensors 12 may include a plurality of capacitative sensors. These do not require good contact with the skin. Suitable sensors are disclosed in WO2007/060609 (Philips). The sensors are integrated into a textile item, here the patch of a T-shirt. As many sensors as are required may be used.

Alternative embodiments may accordingly include ten sensors in the standard ECG configuration, or a reduced number of sensors for example five sensors in the so-called EASI lead configuration which can then be used to calculate a derived ECG.

The sensors need not be integrated into a patch, but may also be integrated into a belt, or other item. Some sensors may be integrated into different wearable items. In a particular embodiment, a number of ECG sensors are integrated into a textile item for covering the thorax, for example a T-shirt. This provides a convenient wearable substrate for the ECG sensors.

The accelerometer 44 need not be incorporated into the patch, but may instead be incorporated into the textile item, belt, or even the processor 30 where worn.

The measurement and recordal of sensor data need not include only the factors mentioned above. Where additional data is recorded it may be used as context data for determining whether to sound or supress an alarm.

The patch 10 may include a temperature sensor 16, which may be used to record body temperature fluctuations. In cases where the equipment is used for the nighttime diagnosis of sleep problems, temperature may be a very relevant consideration.

Further, an impedance sensor 17 may be incorporated into the patch, which injects a small current at the thorax and hence measures the resistance. Impedance cardiography may be used to provide information about tissue composition of the thorax and the pump function and mechanical activity (stroke volume and cardiac output) of the heart.

A microphone 18 may be included in the patch. This can be used to measure heart and lung sounds. The microphone may be a piezo microphone. Heart and lung sounds give additional information about the heart and lung function, including valve sounds of the heart.

The accelerometer 44 may be a two axis or three axis accelerometer.

The system described has particular application to elderly care, cardiac rehabilitation and blood pressure Holter monitoring.

## Claims

1. Apparatus for detecting impending vasovagal syncopes, comprising:
- a plurality of wearable sensors (8) for arrangement on the human body of a patient, the sensors including a plurality of ECG sensors (12), one of the sensors being a heart sensor for arrangement in the vicinity of the heart when the sensors are arranged on the body;
- a remote pulse sensor (22) for arrangement on the human body away from the heart; and
- a processing unit (30) arranged to receive data from the ECG sensors (12) and the remote pulse sensors, to determine the pulse transit time for a pulse to travel from the heart ECG sensor to the remote pulse sensor, and to determine the presence or advent of a vasovagal syncope from the pulse transit time.

2. Apparatus according to claim 1,
wherein the remote pulse sensor (22) is on a wristband (20).

3. Apparatus according to claim 1 or 2 wherein
the heart sensor is one of the plurality of ECG sensors (12).

4. Apparatus according to claim 3 arranged to determine the pulse transit time as the time between a predetermined feature of the ECG measured using the heart sensor and the pulse being detected by the remote pulse sensor.

5. Apparatus according to any preceding claim comprising a patch (10) on which the ECG sensors are arranged.

6. Apparatus according to any preceding claim wherein the processing unit is arranged to determine the presence of a vasovagal syncope by comparing the ratio of the pulse transit time and a reference pulse transit time, and determining the presence of a vasovagal sycope when the ratio exceeds a predetermined value.

7. Apparatus according to claim 6 further comprising an accelerometer (14), the apparatus being arranged for measuring activity and posture in dependence of data from the accelerometer, the apparatus being further arranged to determine the reference pulse transit time when the patient is in a stationary and standing position for a predetermined period.

8. Apparatus according to claim 7 wherein the apparatus is further arranged to determine from the accelerometer data if the patient has fallen, the processing unit (3) further comprising a transceiver (42) for contacting emergency services if the patient has fallen,

9. Apparatus according to claim 7 wherein the apparatus is further arranged to sound an alarm when the ratio exceeds the predetermined value provided that based on accelerometer data it is determined that predetermined conditions for activity and posture exist.

10. A method of processing data from a subject to detect impending vasovagal syncopes, comprising:
- arranging a plurality of sensors over the body of the subject, the sensors including at least one heart sensor for arrangement in the vicinity of the heart and a remote pulse sensor (22) on the human body away from the heart;
- receiving data from the sensors;
- determining the pulse transit time for a pulse to travel from the heart sensor to the remote pulse sensor; and
- comparing the ratio of the pulse transit time and a reference pulse transit time.

11. A method according to claim 10, including
- measuring the pulse transit time when the subject is standing still for a predetermined period;
- using the measured pulse transit time as the reference pulse transit time.

12. A method according to claim 10 or 11, further comprising:
- if the ratio of the pulse transit time and a reference pulse transit time exceeds a predetermined value, determining using an accelerometer if predetermined conditions exist.

13. A method according to claim 12, further comprising sounding an alarm if the ratio of the pulse transit time and a reference pulse transit time exceeds a predetermined value and the predetermined conditions exist.

## Patentansprüche

1. Gerät zum Erkennen von bevorstehenden vasovagalen Synkopen, das Folgendes umfasst:
- eine Vielzahl von tragbaren Sensoren (8) zur Anordnung auf dem Körper eines menschlichen Patienten, wobei die Sensoren eine Vielzahl von EKG-Sensoren (12) umfassen, wobei einer der Sensoren ein Herzsensor zur Anordnung in der Nähe des Herzens ist, wenn die Sensoren auf dem Körper angeordnet werden;
- einen abgesetzten Pulssensor (22) zur Anordnung auf dem menschlichen Körper entfernt von dem Herzen; und
- eine Verarbeitungseinheit (30), die vorgesehen ist, um Daten von den EKG-Sensoren (12) und den abgesetzten Pulssensoren zu empfangen, um die Pulslaufzeit zu ermitteln, die ein Puls benötigt, um von dem Herz-EKG-Sensor zum abgesetzten Pulssensor zu laufen, und um die Anwesenheit oder das Eintreffen einer vasovagalen Synkope anhand der Pulslaufzeit zu ermitteln.

2. Gerät nach Anspruch 1, wobei sich der abgesetzte Pulssensor (22) auf einem Handgelenkband (20) befindet.

3. Gerät nach Anspruch 1 oder 2, wobei der Herzsensor einer der Vielzahl von EKG-Sensoren (12) ist.

4. Gerät nach Anspruch 3, das vorgesehen ist, um die Pulslaufzeit als die Zeit zwischen einem vordefinierten Merkmal des mittels des Herzsensors gemessenen EKGs und dem durch den abgesetzten Pulssensor erkannten Puls zu ermitteln.

5. Gerät nach einem der vorhergehenden Ansprüche, mit einem Pflaster (10), auf dem die EKG-Sensoren angeordnet sind.

6. Gerät nach einem der vorhergehenden Ansprüche, wobei die Verarbeitungseinheit vorgesehen ist, um die Anwesenheit einer vasovagalen Synkope zu ermitteln, indem das Verhältnis der Pulslaufzeit und einer Referenz-Pulslaufzeit verglichen wird und die Anwesenheit einer vasovagalen Synkope ermittelt wird, wenn das Verhältnis einen vorgegebenen Wert überschreitet.

7. Gerät nach Anspruch 6, weiterhin einen Beschleunigungsmesser (14) umfassend, wobei das Gerät vorgesehen ist, um die Aktivität und Haltung in Abhängigkeit von Daten von dem Beschleunigungsmesser zu messen, wobei das Gerät weiterhin angeordnet ist, um die Referenz-Pulslaufzeit zu ermitteln, wenn sich der Patient für eine vorgegebene Zeitdauer in einer stationären und stehenden Position befindet.

8. Gerät nach Anspruch 7, wobei das Gerät weiterhin vorgesehen ist, um anhand der Beschleunigungsmesserdaten zu ermitteln, ob der Patient gestürzt ist, wobei die Verarbeitungseinheit (3) weiterhin einen Transceiver (42) umfasst, um Notdienste zu benachrichtigen, wenn der Patient gestürzt ist.

9. Gerät nach Anspruch 7, wobei das Gerät weiterhin vorgesehen ist, um einen Alarm auszugeben, wenn das Verhältnis den vorgegebenen Wert überschreitet, sofern basierend auf den Beschleunigungsmesserdaten ermittelt wird, dass vorgegebene Bedingungen für Aktivität und Haltung vorliegen.

10. Verfahren zur Verarbeitung von Daten von einen Subjekt, um bevorstehende vasovagale Synkopen zu erkennen, wobei das Verfahren Folgendes umfasst:
- Anordnen einer Vielzahl von Sensoren auf dem Körper des Subjekts, wobei die Sensoren mindestens einen Herzsensor zur Anordnung in der Nähe des Herzens und einen abgesetzten Pulssensor (22) auf dem menschlichen Körper entfernt von dem Herzen umfassen;
- Empfangen von Daten von den Sensoren;
- Ermitteln der Pulslaufzeit zu ermitteln, die ein Puls benötigt, um von dem Herzsensor zum abgesetzten Pulssensor zu laufen; und
- Vergleichen des Verhältnisses der Pulslaufzeit und einer Referenz-Pulslaufzeit.

11. Verfahren nach Anspruch 10, das Folgendes umfasst:
- Messen einer Pulslaufzeit, wenn das Subjekt für eine vorgegebene Zeitdauer stillsteht;
- Verwenden der gemessenen Pulslaufzeit als Referenz-Pulslaufzeit.

12. Verfahren nach Anspruch 10 oder 11, das weiterhin Folgendes umfasst:
- wenn das Verhältnis der Pulslaufzeit und der Referenz-Pulslaufzeit einen vorgegebenen Wert überschreitet, Ermitteln mit Hilfe eines Beschleunigungsmessers, ob vorgegebene Bedingungen vorliegen.

13. Verfahren nach Anspruch 12, weiterhin umfassend das Ausgeben eines Alarms, wenn das Verhältnis der Pulslaufzeit und der Referenz-Pulslaufzeit einen vorgegebenen Wert überschreitet und die vorgegebenen Bedingungen vorliegen.

## Revendications

1. Appareil pour détecter des syncopes vaso-vagotoniques imminentes, comprenant :
- une pluralité de capteurs portables (8) à disposer sur le corps humain d'un patient, les capteurs comprenant une pluralité de capteurs d'ECG (12), l'un des capteurs étant un capteur cardiaque à disposer à proximité du coeur, quand lesdits capteurs sont agencés sur le corps ;
- un capteur de pulsations distant (22) à disposer sur le corps humain loin du coeur ; et
- une unité de traitement (30) disposée de façon à recevoir des données des capteurs d'ECG (12) et des capteurs de pulsations distants, pour déterminer le temps de transit des pulsations pour qu'une pulsation passe du capteur d'ECG cardiaque au capteur de pulsations distant, et pour déterminer la présence ou l'imminence d'une syncope vaso-vagotonique d'après le temps de transit de la pulsation.

2. Appareil selon la revendication 1, dans lequel le capteur de pulsation distant (22) est sur un serre-poignet (20).

3. Appareil selon la revendication 1 ou 2, dans lequel le capteur cardiaque est un de la pluralité des capteurs d'ECG (12).

4. Appareil selon la revendication 3, disposé de façon à déterminer le temps de transit de la pulsation comme le temps entre une caractéristique prédéterminée de l'ECG, mesuré en utilisant le capteur cardiaque et la pulsation détectée par le capteur de pulsation distant.

5. Appareil selon l'une quelconque des revendications précédentes, comprenant un patch (10) sur lequel les capteurs d'ECG sont disposés.

6. Appareil selon l'une quelconque des revendications précédentes, dans lequel l'unité de traitement est disposée de façon à déterminer la présence d'une syncope vaso-vagotonique, en comparant le rapport du temps de transit de la pulsation et d'un temps de transit de pulsation de référence, et en déterminant la présence d'une syncope vaso-vagotonique quand le rapport dépasse une valeur prédéterminée.

7. Appareil selon la revendication 6, comprenant en outre un accéléromètre (14), l'appareil étant disposé de façon à mesurer l'activité et la posture en fonction de données provenant de l'accéléromètre, l'appareil étant en outre disposé pour déterminer le temps de transit de la pulsation de référence quand le patient est en position stationnaire et debout pendant un laps de temps prédéterminé.

8. Appareil selon la revendication 7, dans lequel l'appareil est en outre disposé de façon à déterminer, d'après les données de l'accéléromètre, si le patient est tombé, l'unité de traitement (3) comprenant en outre un émetteur-récepteur (42) pour contacter les services d'urgence si le patient est tombé.

9. Appareil selon la revendication 7, dans lequel l'appareil est en outre disposé de façon à faire retentir une alarme quand le rapport dépasse la valeur prédéterminée à condition que, sur la base des données de l'accéléromètre, il soit déterminé que les conditions prédéterminées pour l'activité et la posture existent.

10. Procédé de traitement de données provenant d'un sujet pour détecter des syncopes vaso-vagotoniques imminentes, comprenant :
- la disposition d'une pluralité de capteurs sur le corps du sujet, les capteurs comprenant au moins un capteur cardiaque à disposer à proximité du coeur et un capteur de pulsation distant (22) sur le corps humain, loin du coeur ;
- la réception de données des capteurs ;
- la détermination du temps de transit de la pulsation pour qu'une pulsation passe du capteur cardiaque au capteur de pulsation distant ; et
- la comparaison du rapport du temps de transit de la pulsation et d'un temps de transition de pulsation de référence.

11. Procédé selon la revendication 10, comprenant :
- la mesure du temps de transit de la pulsation quand le sujet est debout sans bouger pendant un laps de temps prédéterminé ;
- l'utilisation du temps de transit de la pulsation mesuré comme temps de transit de la pulsation de référence.

12. Procédé selon la revendication 10 ou 11, comprenant en outre :
- si le rapport du temps de transit de la pulsation et d'un temps de transit de pulsation de référence dépasse une valeur prédéterminée, la détermination de l'utilisation d'un accéléromètre si des conditions prédéterminées existent.

13. Procédé selon la revendication 12, comprenant en outre l'activation d'une alarme sonore si le rapport du temps de transit de la pulsation et d'un temps de transit de la pulsation de référence dépasse une valeur prédéterminée et si les conditions prédéterminées existent.
